# EUROPEAN PATENT APPLICATION

(11) **EP 2 246 036 A1**
(43) Date of publication of application: **03.11.2010**
(21) Application number: 09005821.5
(22) Date of filing: 27.04.2009
(51) Int. Cl.: A61K 8/44, A61K 8/46, A61K 8/60, A61K 8/64, A61K 8/86, A61Q 5/12, A61Q 5/02, A61K 8/73, A61K 8/35, A61K 8/81, A61K 8/33, A61K 8/92, A61K 8/365, A61K 8/891

(54) **Aqueous cleansing composition**

(71) Applicant: KPSS-Kao Professional Salon Services GmbH, 64297 Darmstadt (DE)
(72) Inventor: Hoffmann, Martin, 64673 Zwingenberg (DE); Grit, Mustafa, Dr., 64579 Gernsheim (DE)
(74) Representative: Grit, Mustafa

(57) **Abstract**

The present invention is related to an aqueous cleansing composition for keratin fibres especially human hair with excellent conditioning effects. Accordingly, first object of the present invention is an aqueous cleansing composition for keratin fibres, especially human hair, comprising at least one foaming surfactant selected from anionic, non-ionic and amphoteric ones and at least one dipeptide. The most preferred dipeptide is carnosine and is of ß-alanin and L-histidine.

## Description

The present invention is related to an aqueous cleansing composition for keratin fibres especially human hair with excellent conditioning effects.

Aqueous cleansing compositions have been known for many years. Many patent applications and scientific publications deal with such compositions aiming at aqueous cleansing and especially improved conditioning effects on hair. Although the prior art developed quite extensively, there is still need for further improvements especially in natural hair feeling, improved grip and improved manageability.

The present invention aims at providing an aqueous cleansing composition having excellent cleansing and conditioning effects especially in terms of natural hair feeling, improved grip and improved manageability.

The present inventors have surprisingly found out that an aqueous cleansing composition based on at least one foaming surfactant and comprising further at least one dipeptide cleanses and conditions hair excellently. It has been observed that aqueous cleansing composition has excellent foaming properties in terms of foam volume and creaminess and hair feels smooth and thoroughly rinseable during usage and hair feels smooth, naturally soft, has improved grip and manageability in both wet and dry stages. It has, furthermore, been observed that dry hair has improved body and volume and enhanced shine.

Accordingly, first object of the present invention is an aqueous cleansing composition for keratin fibres, especially human hair, comprising at least one foaming surfactant selected from anionic, non-ionic and amphoteric ones and at least one dipeptide.

Further object of the present invention is the use of aqueous cleansing composition for cleansing and conditioning of keratin fibres especially human hair.

With the term dipeptide, compounds with two amino acid moieties are meant. Aqueous cleansing composition of the present invention comprises at least one surfactant selected from anionic, non-ionic and amphoteric surfactants at a concentration range of 5 to 50%, preferably 5 to 40% and more preferably 7.5 to 30%, and most preferably 10 to 25% by weight, calculated to the total composition.

In an embodiment of the present invention, aqueous cleansing composition of the present invention comprises at least one anionic, at least one nonionic surfactant. More preferably, the composition further comprises additionally at least one amphoteric surfactant.

Anionic surfactants suitable within the scope of the invention are preferably present in an amount from 1 to about 30%, preferably 2 to 20% and most preferably 2 - 15%, and most preferably 2 to 10% by weight, calculated to the total composition.

In principal any anionic surfactant is suitable within the meaning of the present invention. Nonlimiting examples are anionic surfactants of the sulfate, sulfonate, carboxylate and alkyl phosphate type, especially, of course, those customarily used in shampoo compositions, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates constituting mild, skin-compatible detergents.

Additional anionic surfactants useful within the scope of the invention are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof of the formula

R₃-(C₂H₄O)ₙ-O-CH₂COOX,

wherein R₃ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted, as well as alkyl amido polyether carboxylic acids of the general formula 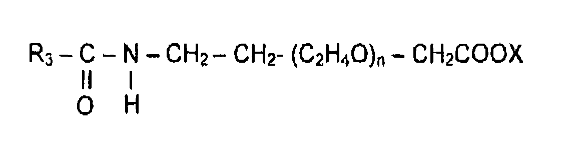 wherein R₃ and X have the above meanings, and n is in particular a number from 1 to 10, preferably 2.5 to 5.

Such products have been known for some time and are on the market, for example, under the trade name "AKYPO^{®}" and "AKYPO-SOFT^{®}".

Also useful are C₈-C₂₀-acyl isethionates, alone or in admixture with other anionic surfactants, as well as sulfofatty acids and the esters thereof.
It is also possible to use mixtures of several anionic surfactants, for example an ether sulfate and a polyether carboxylic acid or alkyl amidoether carboxylic acid.

Further suitable anionic surfactants are also C₈-C₂₂-acyl aminocarboxylic acids or the water-soluble salts thereof. Especially preferred is N-lauroyl glutamate, in particular as sodium salt, as well as, for example, N-lauroyl sarcosinate, N-C₁₂-C₁₈-acyl asparaginic acid, N-myristoyl sarcosinate, N-oleoyl sarcosinate, N-lauroyl methylalanine, N-lauroyl lysine and N-lauroyl aminopropyl glycine, cocoyl glutamate preferably in form of the water-soluble alkali or ammonium, in particular the sodium salts thereof, preferably in admixture with the above-named anionic surfactants.

The most preferred anionic surfactants within the meaning of the present invention are those of alkyl ether sulphates such as lauryl ether sulphate and aminocarboxylic acids such as lauroyl glutamate sodium salt.

Further surfactants in the shampoo compositions according to the invention are nonionic surfactants especially in admixture with anionic surfactants. Especially suited are alkyl polyglucosides of the general formula

R₄-O-(R₆O)ₙ-Zₓ,

wherein R₄ is an alkyl group with 8 to 18 carbon atoms, R₅ is an ethylene or propylene group, Z is a saccharide group with 5 to 6 carbon atoms, n is a number from 0 to 10 and x is a number between 1 and 5.

These alkyl polyglucosides have recently become known in particular as excellent skin-compatible, foam improving agents in liquid detergents and body cleansing compositions, and are present in an amount from about 1 % to 15 %, in particular from 1 % to 10 % by weight, calculated to the total composition.

Mixtures of anionic surfactants and alkyl polyglucosides as well as the use thereof in liquid body cleansing compositions are already known, for example, from EP-A 70 074. The alkyl polyglucosides disclosed therein are basically also suited within the scope of the present invention; as well as the mixtures of sulfosuccinates and alkyl polyglucosides disclosed in EP-A 358 216.

Further nonionic surfactant components may be present, for example, long-chain fatty acid dialkanolamides, such as coco fatty acid diethanolamide and myristic fatty acid diethanolamide, which can also be used as foam enhancers, preferably in amounts from about 1 % to about 5 % by weight.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}", as well as fatty alcohol ethoxylates.

Further suitable nonionic surfactants are amineoxides which may be present in an amount from 0.25 % to 5 % by weight, calculated to the total composition. Such amineoxides are state of the art, for example C₁₂-C₁₈- alkyl dimethyl amineoxides such as lauryl dimethyl amineoxide, C₁₂-C₁₈- alkyl amidopropyl or -ethyl amineoxides, C₁₂-C₁₈ -alkyl di(hydroxyethyl) or (hydroxypropyl) amineoxides, or also amineoxides with ethyleneoxide and/or propyleneoxide groups in the alkyl chain. Such amineoxides are on the market, for example, under the trade names "Ammonyx^{®}", "Aromox^{®}" or "Genaminox^{®}".

Further nonionic surfactants useful in the compositions according to invention are C₁₀-C₂₂-fatty alcohol ethoxylates at a concentration of 0.5 to 10%, preferably 0.5 to 5% by weight, calculated to total composition. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":

The average degree of ethoxylation thereby ranges between about 2.5 and about 25, preferably about 10 and about 20.

The most preferred non-ionic surfactants are alkyl polyglucosides such as decyl, cocoyl polyglucoside and ethoxylated fatty alcohols such as laureth-16.

As further surfactant component, the composition according to the invention also comprises amphoteric surfactants, for example in an amount from about 0.5 % to about 15 %, preferably from about 1 % to about 10 %, by weight, calculated to the total composition. It has especially been found out that addition of amphoteric surfactants enhances foam feeling in terms of creaminess, foam volume and as well as skin compatibility is improved. For achieving milder formulations, anionic surfactant, especially of sulphate types, to amphoteric surfactant ratio should be in the range of 10:1 to 1:1, preferably 5:1 to 1:1.

Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also proven suitable.

In detail, it is possible to use betaines of the structure 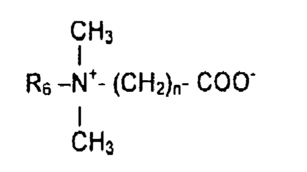 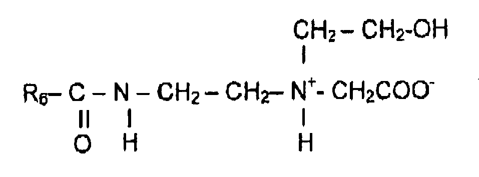 wherein R₆ is a C₈-C₁₈-alkyl group and n is 1 to 3;
sulfobetaines of the structure 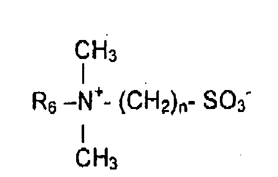 wherein R₆ and n are same as above;
and amidoalkyl betaines of the structure 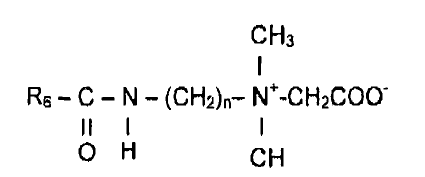 wherein R₆ and n are same as above.

The most preferred amphoteric surfactants are alkyl betaines such as lauryl betaine and alkyl amido betaines such as cocamidopropyl betaine.

In the preferred embodiment of the present invention, aqueous cleansing composition comprises at least one anionic surfactant especially of alkyl ether sulphate type, at least one amphoteric surfactant especially alkyl amido alkyl betaine type and at least one non-ionic surfactant especially an alkyl polyglucoside. In the most preferred form of the present invention, in addition to the above mentioned surfactant the composition comprises additionally acyl amido carboxylic acid surfactant especially sodium lauroyl glutamate.

Aquesous cleansing composition of the present invention comprise at least one dipeptide. The dipeptide compounds according to the present invention comprise two amino acid moieties. In principal, any dipeptide available either natural or synthetic is suitable for the purposes of the present invention. The synthetic ones are preferred. In one of the preferred embodiment of the present invention the amino acid moeities of dipeptide are selected from arginine, tyrosine, valine, tryptophan, alanine, cysteine, glycine, lysine, proline, hydroxyproline and histidine. The dipetides according to the present invention may certainly be of two different amino acids but at the same time two of the same amino acids. In a further preferred embodiment of the present invention, the two amino acid moieties are of different amino acids when one of the amino acid moieties is glycine. More preferably the two amino acid moieties are of two different amino acids.

Non-limiting examples to the suitable dipeptides are the ones commercially available and known with their INCI name as Dipeptide-1, Dipeptide-2, Dipeptide-3, Dipeptide-4, Dipeptide-5, Dipeptide-6, Dipeptide-7, Dipeptide-8, and carnosine. The most preferred is carnosine and is of β-alanin and L-histidine.

Concentration of at least one dipeptide is in the range of 0.01 to 5%, preferably 0.05 to 3% and more preferably 0.1 to 2.5% and most preferably 0.2 to 1.5% by weight calculated to the total composition.

The composition of the present invention comprises hair-conditioning agents. Conditioning agents are selected from oily substances, non-ionic substances, cationic amphiphilic ingredients, cationic polymers or their mixtures.

Oily substances are selected from such as silicone oils, either volatile or non-volatile, natural and synthetic oils. Among silicone oils those can be added to the compositions include dimethicone, dimethiconol, polydimethylsiloxane, DC fluid ranges from Dow Corning, arylated silicones such as phenyl trimethicone or any other silicones with up to 5 aryl, preferably phenyl, group in its molecule, natural oils such as olive oil, almond oil, avocado oil, wheatgerm oil, ricinus oil and the synthetic oils, such as mineral oil, isopropyl myristate, palmitate, stearate and isostearate, oleyl oleate, isocetyl stearate, hexyl laurate, dibutyl adipate, dioctyl adipate, myristyl myristate and oleyl erucate.

Further suitable oily substances are di alkyl ethers of the following general structure

R₁-O-R₂

wherein R₁ and R₂ are same or different saturated or unsaturated and branched or straight alkyl chain with 8 to 24 C atoms and preferably present at a concentration of 0.01 to 5%, more preferably 0.05 to 4%, most preferably 0.1 to 3% by weight, calculated to total composition. Preferably, the R₁ and R₂ of the above general structure are same or different saturated or unsaturated and branched or straight alkyl chain with 8 to 22 C atoms and more preferably 8 to 18 C atoms.

Suitably examples and preferred are dicaprylyl ether, didecyl ether, dilauryl ether, dimyristyl ether, dicetylyl ether, and distearyl ether. Especially preferred are dicaprylyl ether and distearyl ether.

Non-ionic conditioning agents may be polyols such as glycerin, glycol and derivatives, polyethyleneglycoles known with trade names Carbowax PEG from Union Carbide and Polyox WSR range from Amerchol and polyglycerin.

In one of the preferred form of the present invention, aqueous cleansing composition comprises at least one cationic polymer as conditioning agent. Suitable cationic polymers are those of best known with their CTFA category name Polyquaternium. Typical examples of those are Polyquaternium 1, Polyquaternium 2, Polyquaternium 4, Polyquaternium 5, Polyquaternium 6, Polyquaternium 7, Polyquaternium 8, Polyquaternium 9, Polyquaternium 10, Polyquaternium 11, Polyquaternium 12, Polyquaternium 13, Polyquaternium 14, Polyquaternium 15, Polyquaternium 16, Polyquaternium 17, Polyquaternium 18, Polyquaternium 19, Polyquaternium 20, Polyquaternium 22, Polyquaternium 24, Polyquaternium 27, Polyquaternium 28, Polyquaternium 29, Polyquaternium 30, Polyquaternium 31, Polyquaternium 32, Polyquaternium 33, Polyquaternium 34, Polyquaternium 35 and Polyquaternium 36, Polyquaternium-37, Polyquaternium 39, Polyquaternium 42, Polyquaternium 43, Polyquaternium 44, Polyquaternium 45, Polyquaternium 46, Polyquaternium 47, Polyquaternium 48, Polyquaternium-49, Polyquaternium 50, Polyquaternium 51, Polyquaternium 52, Polyquaternium 53, Polyquaternium 54, Polyquaternium 55, Polyquaternium 56, Polyquaternium 57, Polyquaternium 58, Polyquaternium 59, Polyquaternium 60, Polyquaternium 61, Polyquaternium 62, Polyquaternium 63, Polyquaternium 64, Polyquaternium 65, Polyquaternium 66, Polyquaternium 67, Polyquaternium 68, Polyquaternium 69, Polyquaternium-70, Polyquaternium 71, Polyquaternium 72, Polyquaternium 73, Polyquaternium 74, Polyquaternium 75, Polyquaternium 76, Polyquaternium 77, Polyquaternium 78, Polyquaternium-79, Polyquaternium 80, Polyquaternium 81, Polyquaternium 82, Polyquaternium 83, Polyquaternium 84, Polyquaternium 85, Polyquaternium 86 and Polyquaternium 87.

It has further been found out that especially those of cationic cellulose type polymers known as Polymer JR type from Amerchol such as Polyquaternium 10 or cationic galactomannans such as cationic guar gum known with trade name Jaguar from Rhône-Poulenc which are chemically for example Guar hydroxypropyl trimonium chloride and cationic tara gum and its derivatives known with INCI name Caesalpinia spinosa hydroxypropyltrimonium chloride, are preferred ones. Furthermore, chitosan and chitin can also be included in the compositions as cationic natural polymers. In this context reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

The most preferred cationic polymers are those of cationic cellulose derivatives, cationic guar gum derivatives, cationic Caesalpinia spinosa gum derivatives, polyquaternium 6, polyquaternium 7, polyquaternium 67 and polyquaternium 70.

The cationic polymers also include the quaternized products of graft polymers from organopolysiloxanes and polyethyl oxazolines described in EP-A 524 612 and EP-A 640 643.

Although less preferred, aqueous cleansing compositions of the present invention can comprise additionally one or more cationic surfactant(s) as conditioner presented with the general formula 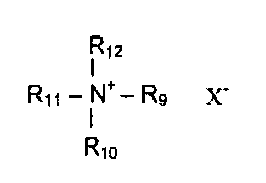 where R₉ is a saturated or unsaturated, branched or non-branched alkyl chain with 8-22 C atoms or

R₁₃CO NH(CH₂)ₙ

where R₁₃ is saturated or unsaturated, branched or non-branclled alkyl chain with 7-21 C atoms and n has value of 1 - 4,
or

R₁₄COO(CH₂)ₙ

where R₁₄ is saturated or unsaturated, branched or non-branched alkyl chain with 7-21 C atoms and n has value of 1 - 4, and

R₁₀ is hydrogen or unsaturated or saturated, branched or non-branched alkyl chain with 1 - 22 C atoms or

R₁₃CONH(CH₂)ₙ

or

R₁₄COO(CH₂)ₙ

where R₁₃, R₁₄ and n are same as above.

R₁₁ and R₁₂ are hydrogen or lower alkyl chain with 1 to 4 carbon atoms which may be substituted with one or two hydroxyl groups, and X is anion such as chloride, bromide, methosulfate.

Typical examples of those ingredients are cetyl trimethly ammonium chloride, stear trimonium chloride, behentrimoinium chloride, stearamidopropyl trimonuim chloride, dioleoylethyl dimethyl ammonium methosulfate, dioleoylethyl hydroxyethylmonium methosulfate.

The compositions according to the invention may also comprise further conditioning substances such as protein hydrolyzates and polypeptides, e.g., keratin hydrolyzates, collagen hydrolyzates of the type "Nutrilan^{R}" or elastin hydrolyzates, as well as also in particular plant protein hydrolyzates, optionally, cationized protein hydrolyzates, e.g., "Gluadin^{R}".

Typical concentration range for any of those conditioners of cationic polymers, silicone oil and derivatives, cationic surfactants and protein hydrolyzates is in the range of 0.01 - 5% by weight, preferably 0.01 - 3.5% by weight, more preferably 0.05 - 2.5% and most preferably 0.1 - 1.5% by weight calculated to the total composition. Most preferred conditioning agents are cationic polymers.

Further conditioning additives are hair conditioning and/or styling polymers. These may be nonionic polymers, preferably alcohol- and/or water-soluble vinyl pyrrolidone polymers, such as a vinyl pyrrolidone homopolymers or copolymers, in particular with vinyl acetate. Useful vinyl pyrrolidone polymers are, e.g., those known by the trade name "Luviskol®", for example, the homopolymers "Luviskol® K 30, K 60 and K 90", as well as the water-or alcohol-soluble copolymers from vinyl pyrrolidone and vinyl acetate, distributed by BASF AG under the trade name "Luviskol® VA 55 respectively VA 64". Further possible nonionic polymers are vinyl pyrrolidone/vinyl acetate/vinyl propionate copolymers such as "Luviskol® VAP 343", vinyl pyrrolidone/(meth)acrylic acid ester copolymers, as well as chitosan derivatives.

Amphoteric polymers are found to be useful in conditioning shampoo composition of the present invention. They are incorporated alone or in admixture with at least one additional cationic, nonionic or anionic polymer, particularly copolymers of N-octyl acrylamide, (meth)acrylic acid and tert.-butyl aminoethyl methacrylate of the type "Amphomer®"; copolymers from methacryl oylethyl betaine and alkyl methacrylates of the type "Yukaformer®", e.g., the butyl methacrylate copolymer "Yukaformer® AM75"; copolymers from monomers containing carboxyl groups and sulfonic groups, e.g., (meth)acrylic acid and itaconic acid, with monomers such as mono- or dialkyl amino alkyl(meth)acrylates or mono- or dialkyl aminoalkyl (meth)acrylamides containing basic groups, in particular amino groups; copolymers from N-octyl acrylamide, methyl methacrylate, hydroxypropyl methacrylate, N-tert.-butyl aminoethyl - methacrylate and acrylic acid, as well as the copolymers known from US-A 3,927,199, are applicable.

Aqueous cleansing composition of the present invention can be transparent as well as pearly. Transparency of the composition is judged by naked eye in a transparent shampoo bottle with a thickness not more than 5 cm. In the case a transparent appearance is wished, the following ingredients are not essential. Pearl-shiny appearance is achieved with those dispersed in aqueous cleansing conditioning compositions in crystalline form, i.e. so called pearl-shine or pearlizing agents. The preferred once are PEG-3 distearate and ethylene glycol distearate. The concentration of those can typically be from 0.1 to 3%, preferably 0.5 to 2% by weight, calculated to the total composition. These compounds are preferably added to the compositions in admixture with anionic, nonionic and/or amphoteric surfactants. Such kinds of mixtures are available commercially.

Solubilizers may be added to the compositions especially when oily substances are chosen as conditioning agents and fragrance oils with highly lipophilic properties. Typical solubilizers may be hydrogenated castor oil known with the trade mark Cremophor CO series from BASF. It should be noted that as well the surfactant mixture can be a good solubilizer for fragrance oils. Typical concentration of the solubilizers can be in the range of 0.01 - 2% by weight, preferably 0.1 - 1% by weight, calculated to total composition.

The aqueous cleansing composition may contain active ingredients selected from UV filters, moisturisers, sequestering agents, and natural ingredients.

The moisturizing agents are selected from panthenol, polyols, such as glycerol, polyethylene glycols with molecular weight 200 to 20,000 and polypropylene glycols. The moisturizing ingredients can be included in the conditioner compositions at a concentration range of 0.01 - 2.5% by weight calculated to the total composition.

Suitable non-limiting examples to the polyethyleneglycols are PEG-800, PEG-2M, PEG-5M, PEG-7M, PEG-9M, PEG-14M, PEG-20M, PEG-25M, PEG-45M, PEG-65M, PEG-90M, PEG-115 and PEG-160M.

Non-limiting suitable examples to polypropylene glycols are PPG-3, PPG-7, PPG-9, PPG-12, PPG-13, PPG-15, PPG-16, PPG-17, PPG-20, PPG-26, PPG-30, PPG-33, PPG-34, PPG-51 and PPG-69.

The sequestering agents are preferably selected from polycarboxy acids. The preferred one is ethylene diamine tetraacetic acid, EDTA. Typical useful concentration range for sequestering agents is of 0.01 - 2.5% by weight calculated to the total composition.

The UV filters are that oil and water soluble ones for the purpose of protecting hair colour. In other words, anionic and nonionic, oily, UV filters are suitably used in the compositions of the present invention. Suitable UV-absorbing substances is are: 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2,4-dihydroxybenzophenone, 2,2',4,4'-tetrahydroxybenzophenone, 2-hydroxy-4-methoxybenzophenone and its 5-sulfonic acid or the sodium salt thereof, 2,2'-dihydroxy-4,4'-dimethoxybenzophenone, 2-hydroxy-5-chlorobenzophenone, 2,2'-dihydroxy-4-methoxybenzophenone, 2,2'-dihydroxy-4,4'-dimethoxy-5,5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof, 3-(4'-methyl benzylidene)-DL-campher, and/or polysilicone- 15. The amount of the UV-absorber ranges typically from about 0.01 % to 2.5%, more preferably from 0.05 % to 1 % by weight, calculated to the total composition.

Natural plant extracts are incorporated usually in an amount of about 0.01 % to about 10 %, preferably 0.05 % to 7.5 %, in particular 0.1 % to 5 % by weight, calculated as dry residue thereof to the total composition. Suitable aqueous (e.g. steam-distilled) alcoholic or hydro-alcoholic plant extracts known **per se** are in particular extracts from leaves, fruits, blossoms, roots, rinds or stems of aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc. Suitable trade products are, for example, various "Extrapone®" products, and "Herbasol^{R} ". Extracts and the preparation thereof are also described in "Hagers Handbuch der pharmazeutischen Praxis", 4th Ed..

In a further preferred embodiment of the present invention composition comprise at least one polyethyleneglycol diester of the general structure

R₇-C(O)(OCH₂CH₂)ₙO(O)C-R₈

wherein R₇ and R₈ is a straight or branched, saturated or unsaturated alkyl chain with 7 to 23 C atoms, preferably with 9 to 21 C atoms and more preferably with 11 to 19 C atoms and most preferably with 11 to 17C atoms and n is 2 to 100 at a concentration of 0.01 to 5%, preferably 0.05 to 4%, more preferably 0.1 to 3% and most preferably 0.2 to 2.5% by weight calculated to total composition.

Compositions of the present invention may comprise further at least one compound according to the formula 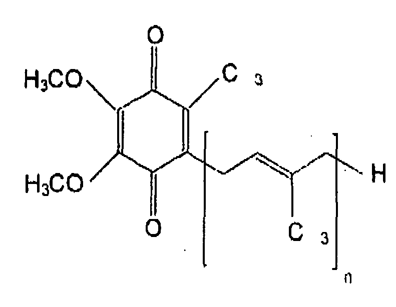 where n is a number between 1 and 10.

The compounds of the above formula are known as Ubiquinone, and also are known as Coenzyme. It should be noted that the compositions of the present invention can certainly comprise more than one ubichinone. Preferred ubichinones are the ones where n is a number between 6 and 10 and especially preferred is Ubichinone 50 where n is 10, also known as Coenzyme Q10. Concentration ubichinone of the above formula in the compositions is from 0.0001 to 1%, preferably from 0.0002 to 0.75%, more preferably from 0.0002 to 0.5% and most preferably from 0.0005 to 0.5% by weight, calculated to total composition.

Aqueous cleansing compositions of the present invention can also comprise synthetic mica as a further shine enhancer.

Use of synthetic mica coated with metal oxide or oxides mainly in decorative cosmetics is disclosed in an international patent application of Sun Chemical Corporation published with a number WO 2005/065632 A1. In the document synthetic mica and coated synthetic mica with at least one metal oxide or oxides is disclosed in detail, the content of the document is included herewith by reference. It also discloses a cleansing composition comprising monoethanolamide surfactant in addition to other surfactants.

Suitable metal oxide or oxides for coating synthetic mica are titanium dioxide, chromium oxide, ferric oxide or mixtures thereof. In the present invention the preferred is synthetic mice coated with titanium dioxide. Such materials are commercially available from Sun Chemical Corporation and known with their INCI names Synthetic Fluorphologopite.

The particle size distribution of synthetic mica coated with a metal oxide or oxides is in the range of 1 to 750 µm, preferably 1 to 250 µm, more preferably 1 to 100 µm and most preferably 20 to 95 µm. The particle sizes referred are relating to the volume particle size distribution meaning that particles found in the coated synthetic mica having volume particle size in the given ranges.

Concentration of synthetic mica coated with at least metal oxide or oxides is from 0.001 to 10%, preferably 0.05 to 7.5%, more preferably 0.1 to 5% and most preferably 0.20 to 2.5% by weight calculated to total composition.

Further, in preferred embodiment of the present invention, compositions for hair comprise at least one direct dye for colouring hair. Suitable direct dyes are of cationic, anionic and neutral nitro dyes. It should be noted that they can also be used in combination with each other. In other words a composition according to present invention can comprise an anionic and a cationic dye as well as an anionic and a nitro dye or a cationic and a nitro dye. Certainly the combination of all three dyestuffs is also possible.

Any cationic direct dye is in principal suitable for the compositions. Examples are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Orange 31, Basic Red 2, Basic Red 12 Basic Red 22, Basic Red 51, Basic Red 76, Basic Violet 1, Basic Violet 2, Basic Violet 3, Basic Violet 10, Basic Violet 14, Basic Yellow 57 and Basic Yellow 87.

Any anionic dye is in principal suitable for the compositions. Suitable examples are such as Acid Black 1. Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

Among those, the preferred anionic dyestuffs are Acid Red 52, Acid Violet 2, Acid Red 33, Acid Orange 4, Acid Red 27 and Acid Yellow 10 and their salts. The most preferred anionic dyes are Acid Red 52, Acid Violet 2, Acid Red 33, Acid Orange 4 and Acid Yellow 10, and their salts

Neutral dyes, so called nitro dyes for shading purposes are also optionally contained in the compositions. Suitable ones are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.6, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No. 15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Concentration of one or more direct dyes in total is in the range of 0.001 to 5% by weight, preferably 0.01 to 4% more preferably 0.05 to 3% and most preferably 0.1 to 2.5% by weight calculated to total composition.

Aqueous cleansing composition of the present invention preferably comprises at least one ethoxylated monoglyceride according to the above general formula ethoxylated monoglyceride according to the general structure 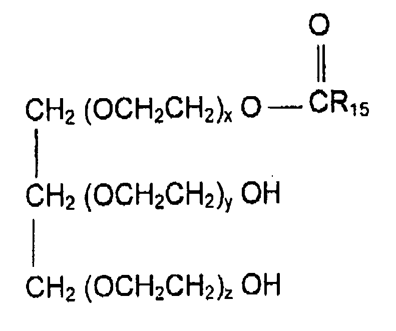 wherein R₁₅ is a saturated or unsaturated and branched or straight alkyl chain with a chain length of 7 to 21 C atoms and x+y+z has a value of 3 to 200. In the preferred embodiment of the present invention R₁₅ is a saturated or unsaturated and branched or straight alkyl chain with a chain length of 11 to 17 C atoms, more preferably 13 to 17 C atoms and most preferably 15 to 17 C atoms and x+y+z has preferably a value of 10 to 150, more preferably 20 to 100 and most preferably 40 to 90.

Ethoxalted monogylcerides are known for their thickening ability in the area of hair cleansing compositions. For example WO 03/ 063818 A1 discloses ethoxylated glycerides as thickening agents in combination with ethoxylated fatty alcohol and ethoxylated partial gylcerides. WO 2004/024110 A1 and WO 03/013467 A1 disclose cleansing compositions comprising an ethoxylated monogylceride.

Non-limiting suitable examples of ethoxylated monoglycerides are PEG-6 glyceryl caprate, PEG-3 glyceryl cocoate, PEG-7 glyceryl cocoate, PEG-30 glyceryl cocoate, PEG-40 glyceryl cocoate, PEG-78 glyceryl cocoate, PEG-80 glyceryl cocoate, PEG-3 glyceryl isostearate, PEG-5 glyceryl isostearate, PEG-6 glyceryl isostearate, PEG-8 glyceryl isostearate, PEG-9 glyceryl isostearate, PEG-10 glyceryl isostearate, PEG-15 glyceryl isostearate, PEG-20 glyceryl isostearate, PEG-25 glyceryl isostearate, PEG-30 glyceryl isostearate, PEG-40 glyceryl isostearate, PEG-50 glyceryl isostearate, PEG-60 glyceryl isostearate, PEG-90 glyceryl isostearate, PEG-7 glyceryl laurate, PEG-8 glyceryl laurate, PEG-12 glyceryl laurate, PEG-15 glyceryl laurate, PEG-20 glyceryl laurate, PEG-23 glyceryl laurate, PEG-30 glyceryl laurate. PEG-5 glyceryl oleate, PEG-10 glyceryl oleate, PEG-15 glyceryl oleate, PEG-20 glyceryl oleate, PEG-26 glyceryl oleate, PEG-30 glyceryl oleate, PEG-15 glyceryl ricinoleate, PEG-20 glyceryl ricinoleate, PEG-5 glyceryl sesquioleate, PEG-7 glyceryl soyate, PEG-30 glyceryl soyate, PEG-5 glyceryl stearate, PEG-10 glyceryl stearate, PEG-15 glyceryl stearate, PEG-20 glyceryl stearate, PEG-25 glyceryl stearate, PEG-30 glyceryl stearate, PEG-40 glyceryl stearate, PEG-60 glyceryl stearate, PEG-120 glyceryl stearate, PEG-200 glyceryl stearate, PEG-28 glyceryl tallowate, PEG-80 glyceryl tallowate, PEG-82 glyceryl tallowate, PEG-130 glyceryl tallowate and PEG-200 glyceryl tallowate.

Among the ethoxylated monogylcerides, with fatty acid chain of laurate, isostearate, oleate and stearate are preferred. More preferred are with fatty acid chain of isostearate, oleate and stearate. The most preferred are with fatty acid chain of isostearate and stearate. Especially preferred is with fatty acid chain of isostearate.

The especially preferred ethoxylated monoglyceride is PEG-90 glyceryl isostearate which is available from Zschimmer & Schwarz under the trade name Oxetal VD 92.

Concentration of ethoxylated monoglyceride in the compositions of the present invention is in the range of 0.1 to 20%, preferably 0.25 to 15%, more preferably 0.5 to 10% and most preferably 1 to 7.5% by weight, calculated to total composition. Aqueous cleansing compositions of the present invention can be in the form of conventional liquid thickened shampoo, as well in the form of ready to use foam, delivered either from a pump-foamer or from an aerosol bottle. In the case that an aerosol foam preparation is preferred, propellant gas must be added to the formulation. The suitable propellant gasses are carbondioxide, dimethylether, alkanes such as butane propane and halogenated alkanes and/or their mixtures.

The viscosity of the aqueous cleansing compositions according to the invention is in the range of 500 and about 20,000 mPa.s at 20°C, preferably 1,000 to 10,000, in particular 1,500 to 7,000 mPa.s at 20°C, measured with Brookfield or Höppler viscosimeters at a shear rate of 10 sec⁻¹.

Viscosity of shampoo compositions can be adjusted with known viscosity enhancers. The preferred ones are monoglycerides such as glyceryl laurate, oleate, and PEG-18 glyceryl oleate/cocoate known with the trade names Antil^{R} 171 and PEG-160 sorbitan triisostearate known with a trade name Rheodol^{R}. It should be noted that in the case that a composition are delivered in the form of a foam from a pump-foamer and/or aerosol can, those compositions should not be thickened and have a viscosity value not more than 500 mPa.s, more preferably 250 mPa.s measured as mentioned above at room temperature.

It is self-understood that the shampoos according to the invention may comprise other substances customarily used in such compositions such as preservatives, fragrances.

The pH of the compositions according to the present invention is suitably between 2 and 8.0, preferably in the range of 2.5 to 7.0, more preferably 3 to 6.5 and most preferably 4 to 5.5 measured at ambient temperature with a suitable pH meter.

pH of the compositions is adjusted with acidic and alkaline compounds. Acidic compounds can be inorganic and organic acid or their mixtures. Nonlimiting suitable examples are citric acid, lactic acid, glycolic acid, hydroxyacrylic acid, glyceric acid, malic acid and tartaric acid and of the dicarboxylic acids are malonic acid, succinic acid, glutaric acid, adipic acid, maleic acid, fumaric acid and phtalic acid. Alkaline compounds such as sodium hydroxide can be used to adjust the pH of the compositions.

The following examples are to illustrate the invention, but not to limit. The compositions according to the invention are prepared by mixing the individual components in water, whereby it is also possible to use pre-mixtures of various ingredients.

### Example 1

| | % by weight |
|---|---|
| Sodium lauryl ether sulphate | 8.0 |
| Coco glucoside | 3.0 |
| Cocamidopropyl betaine | 3.0 |
| Sodium lauroyl glutamate | 1.0 |
| Carnosine | 0.5 |
| Polyquaternium-10 | 1.0 |
| Citric acid/sodium hydroxide | q.s. to pH 5.5 |
| Preservative, fragrance Water | q.s to 100 |

For the comparative purposes the above composition was also prepared without carnosine. It was replaced with water.

The performance of the above example was compared to the comparative composition in a half side test with 10 volunteers. Hair of the volunteer was divided into 2 and washed with example 1 and comparative composition using according to hair length 4 to 6 g of the product. After rinsing, both sides were evaluated by at least 2 hair dressers and by the volunteer in towel dried and dry state. It was found that the side washed with example 1 had significantly better combability, had more shine, grip and felt softer and more natural upon touching. The preferences were generally 9 to 1 and for soft and natural felling it was 10/0.

Similar results were observed with the examples below.

### Example 2

| | % by weight |
|---|---|
| Sodium lauryl ether sulphate | 8.0 |
| Laureth - 16 | 4.0 |
| Cocamidopropyl betaine | 3.0 |
| Sodium lauroyl glutamate | 1.0 |
| Polyquaternium-7 | 1.0 |
| Carnosine | 0.3 |
| Lactic acid/sodium hydroxide | q.s. to pH 5.2 |
| Preservative, fragrance | q.s |
| Water | to 100 |

The above composition improves combability, shine, elasticity,softness and body of hair.

### Example 3

| | | % by weight |
|---|---|---|
| Sodium lauryl ether sulphate | | 1.0 |
| Sodium lauryl ether carboxylate | | 9.0 |
| Laureth - 16 | | 3.0 |
| Cocoyl betaine | | 2.0 |
| Sodium lauroyl glutamate | | 2.0 |
| Polyquaternium-6 | | 0.5 |
| Carnosine | 0.5 | |
| Ubiquinone | | 0.1 |
| PEG-90 glyceryl isostearate | | 4.0 |
| PEG-45M | | 0.5 |
| Benzophenone-3 | | 0.3 |
| Malic acid/sodium hydroxide | | q.s. to pH 5.0 |
| Preservative, fragrance | | q.s |
| Water | | to 100 |

The above composition improves hair volume, gives hair more elasticity. It has excellent foam creaminess.

### Example 4

| | % by weight |
|---|---|
| Sodium lauryl ether sulphate | 8.0 |
| Coco glucoside | 3.0 |
| Cocoamphoacetate | 4.0 |
| Polyquaternium-7 | 0.8 |
| Carnosine | 0.5 |
| PEG-4 diisostearylether | 0.8 |
| PEG-25M | 1.2 |
| Basic red 51 | 0.1 |
| Malic acid/sodium hydroxide | q.s. to pH 5.5 |
| Preservative, fragrance | q.s |
| Water | to 100 |

Above composition gives hair a red shine, in addition to the excellent softness.

### Example 5

| | % by weight |
|---|---|
| Sodium lauryl ether sulfate | 9.0 |
| Laureth - 16 | 3.0 |
| Cocoyl betaine | 2.0 |
| Sodium lauroyl glutamate | 2.0 |
| Guarhydroxypropyltrimonium chloride | 1.0 |
| Carnosine | 0.3 |
| Basic yellow 87 | 0.08 |
| Basic red 51 | 0.001 |
| Citric acid/sodium hydroxide | q.s. to pH 5.0 |
| Preservative, fragrance | q.s |
| Water | to 100 |

Increase of volume and an excellent golden blonde shine was observed on light blond hair.

### Example 6

| | % by weight |
|---|---|
| Sodium lauryl ether sulfate | 9.0 |
| Decyl glucoside | 3.0 |
| Cocoyl betaine | 2.0 |
| Sodium lauroyl glutamate | 2.0 |
| Hydroxypropylguarhydroxy-propyltrimonium chloride | 1.0 |
| Carnosine | 0.6 |
| PEG-25M | 1.0 |
| Wheatgerm oil | 0.1 |
| Basic red 51 | 0.1 |
| Basic orange 31 | 0.05 |
| Basic red 76 | 0.1 |
| Citric acid/sodium hydroxide | q.s. to pH 5.0 |
| Preservative, fragrance | q.s |
| Water | to 100 |

Increase of volume and an excellent red shine and improved softness were observed on medium blond hair.

### Example 7

| | % by weight |
|---|---|
| Sodium lauryl ether sulfate | 9.0 |
| Cocoyl glucoside | 3.0 |
| Cocoyl betaine | 2.0 |
| Sodium lauroyl glutamate | 2.0 |
| Polyquaternium-10 | 1.0 |
| PEG-90 glyceryl isostearate | 3.5 |
| Carnosine | 0.5 |
| Dicetylether | 1.0 |
| Carbopol Aqua CC | 5.0 |
| Synthetic fluorphologopite | 0.5 |
| Citric acid/sodium hydroxide | q.s. to pH 4.7 |
| Preservative, fragrance | q.s |
| Water | to 100 |

| | |
|---|---|
| *: Synthetic fluorphologopite used is commercially available from Sun Chemical Corporation under the trade name SunShine Glitter White with a particle size distribution in the range of 20 to 95 µm. | |

### Example 8

| | % by weight |
|---|---|
| Sodium lauryl ether sulfate | 10.0 |
| Decyl glucoside | 2.0 |
| Cocoyl betaine | 2.0 |
| Sodium lauroyl glutamate | 2.0 |
| Polyquaternium-67 | 0.5 |
| Dimethicone | 0.5 |
| Carnosine | 0.5 |
| PEG-45M | 0.7 |
| Citric acid/sodium hydroxide | q.s. to pH 5.0 |
| Preservative, fragrance | q.s |
| Water | to 100 |

Above shampoo was found to be excellent in improving softness and giving hair more grip in a half side test carried out in a similar way as described above for example 1.

### Example 9

| | % by weight |
|---|---|
| Sodium lauryl ether sulphate | 8.0 |
| Coco glucoside | 2.0 |
| Cocamidopropyl betaine | 4.0 |
| Sodium lauroyl glutamate | 2.0 |
| Trimethyl pentaphenyl trisiloxane | 0.3 |
| Polyquaternium-87 | 1.0 |
| PEG-120 glyceryl stearate | 3.0 |
| PPG-9 | 1.7 |
| Distearyl ether | 0.3 |
| Carnosine | 0.6 |
| Citric acid/sodium hydroxide | q.s. to pH 5.2 |
| Preservative, fragrance | q.s |
| Water | to 100 |

Above shampoo was found to be excellent in enhancing softness and grip of hair. Additionally it improves combability and showed excellent shine enhancing effect.

### Example 10

| | % by weight |
|---|---|
| Sodium lauryl ether sulphate | 8.0 |
| Laureth - 16 | 5.0 |
| Cocamidopropyl betaine | 4.0 |
| Polyquaternium-10 | 1.0 |
| PEG-90 glyceryl isostearate | 2.5 |
| Distearyl ether | 0.3 |
| PEG-45 M | 0.6 |
| Carnosine | 0.3 |
| Citric acid/sodium hydroxide | q.s. to pH 5.2 |
| Preservative, fragrance | q.s |
| Water | to 100 |

The above composition increases hair volume, improves combability, grip and shine.

### Example 11

| | % by weight |
|---|---|
| Sodium lauryl ether carboxylate | 9.0 |
| Decyl glucoside | 3.0 |
| Cocoyl betaine | 2.0 |
| Sodium lauroyl glutamate | 2.0 |
| Polyquaternium-6 | 0.5 |
| Dimethicone | 0.5 |
| Carnosine | 0.3 |
| Distearyl ether | 0.3 |
| PEG-45 M | 0.6 |
| Citric acid/sodium hydroxide | q.s. to pH 5.0 |
| Preservative, fragrance | q.s |
| Water | to 100 |

The above composition improves hair softness, gives hair more elasticity and shine

### Example 12

| | % by weight |
|---|---|
| Sodium lauryl ether sulphate | 8.0 |
| Cocoyl polyglucoside | 3.0 |
| Cocoamphoacetate | 4.0 |
| Cocoyl betaine | 1.0 |
| Guarhydroxypropyl-Trimonium chloride | 0.8 |
| Dimethicone | 0.5 |
| Distearyl ether | 0.3 |
| PEG-45 M | 0.6 |
| PPG-7 | 0.9 |
| Carnosine | 0.3 |
| Basic red 51 | 0.1 |
| Basic red 76 | 0.1 |
| Citric acid/sodium hydroxide | q.s. to pH 5.5 |
| Preservative, fragrance | q.s |
| Water | to 100 |

Above composition gives hair a red shine, in addition to the improved grip and softness.

### Example 13

| | % by weight |
|---|---|
| Sodium lauryl ether sulfate | 9.0 |
| Cocoyl glucoside | 3.0 |
| Cocamidopropyl betaine | 2.0 |
| Sodium lauroyl glutamate | 2.0 |
| Guarhydroxypropyltrimonium chloride | 1.0 |
| Distearyl ether | 0.3 |
| PEG-45 M | 0.6 |
| Carnosine | 0.2 |
| Basic yellow 87 | 0.10 |
| Basic red 76 | 0.02 |
| Basic brown 16 | 0.03 |
| Citric acid/sodium hydroxide | q.s. to pH 5.0 |
| Preservative, fragrance | q.s |
| Water | to 100 |

Increase of volume and an excellent golden blonde shine was observed on light blond hair.

### Example 14

| | % by weight |
|---|---|
| Sodium lauryl ether sulfate | 9.0 |
| Laureth - 16 | 3.0 |
| Cocoyl betaine | 2.0 |
| Sodium lauroyl glutamate | 2.0 |
| Guarhydroxypropyltrimonium chloride | 1.0 |
| PEG-120 glyceryl stearate | 1.8 |
| Distearyl ether | 0.3 |
| PEG-45 M | 0.6 |
| Carnosine | 0.6 |
| Basic red 51 | 0.1 |
| Basic orange 31 | 0.05 |
| Citric acid/sodium hydroxide | q.s. to pH 5.0 |
| Preservative, fragrance | q.s |
| Water | to 100 |

Increase of volume and an excellent red shine were observed on medium blond hair in addition to improved softness.

## Claims

1. Aqueous cleansing composition for keratin fibres especially for human hair comprising at least one surfactant selected from anionic, non-ionic and amphoteric ones **characterised in that** it comprises at least one dipeptide.

2. Composition according to claim 1 **characterized in that** at least one dipeptide is selected from synthetic or natural ones.

3. Composition according to any of the preceding claims **characterized in that** the amino acid moeties of dipeptide are selected from arginine, tyrosine, valine, tryptophan, alanine, cysteine, glycine, lysine, proline, hydroxyproline and histidine and preferably contains two different amino acid moieties when one of the amino acid moieties is glycine and more preferably both aminoacid moieties are of different amino acids.

4. Composition according to any of the preceding claims **characterized in that** it comprises at least one dipeptide at a concentration of 0.01 to 5% by weight calculated to total composition.

5. Composition according to any of the preceding claims **characterized in that** at least one dipeptide is selected from Dipeptide-1, Dipeptide-2, Dipeptide-3, Dipeptide-4, Dipeptide-5, Dipeptide-6, Dipeptide-7, Dipeptide-8, and carnosine.

6. Composition according to any of the preceding claims **characterized in that** at least one dipeptide is carnosine.

7. Composition according to any of the preceding claims **characterised in that** it comprises at least one anionic surfactant and at least one non-ionic surfactant.

8. Composition according to claim 7 **characterised in that** it comprises additionally at least one amphoteric surfactant.

9. Composition according to any of the preceding claims **characterised in that** it comprises at least one conditioning agent.

10. Composition according to claim 9 **characterised in that** conditioning agent is a cationic polymer.

11. Composition according to any of the preceding claims **characterised in that** it comprises at least one UV filter.

12. Composition according to any of the preceding claims **characterised in that** it comprises at least one direct dye.

13. Composition according to claim 12 **characterised in that** it comprises at least one cationic dye as a direct dye.

14. Composition according to any of the preceding claims **characterised in that** it comprises one or more compounds of solubilizers, pearlizing agents, polyol, chelating agent, thickener, natural extract, natural oil, silicone, ubichinone.

15. Use of a composition according to claims 1 to 14 for cleansing and conditioning keratin fibres especially human hair.
